# EUROPÄISCHE PATENTSCHRIFT

(11) **EP 1 098 866 B1**
(45) Veröffentlichungstag und Bekanntmachung des Hinweises auf die Patenterteilung: **11.02.2004**
(21) Anmeldenummer: 99934577.0
(22) Anmeldetag: 06.07.1999
(51) Int. Cl.: C07C 51/15, C07C 63/70, C07D 207/327, C07C 317/44

(54) **VERFAHREN ZUR HERSTELLUNG VON ORTHO-ALKYLIERTEN BENZOESÄUREDERIVATEN**
METHOD FOR PRODUCING ORTHO-ALKYLATED BENZOIC ACID DERIVATIVES
PROCEDE DE PRODUCTION DE DERIVES D'ACIDE BENZOIQUE ORTHO-ALKYLES

(30) Priorität: 23.07.1998 DE 19833118
(43) Veröffentlichungstag der Anmeldung: 16.05.2001
(73) Patentinhaber: MERCK PATENT GmbH, 64293 Darmstadt (DE)
(72) Erfinder: BARTMANN, Ekkehard, D-64390 Erzhausen (DE); STEIN, Ingeborg, D-63110 Rodgau (DE)
(86) Internationale Anmeldenummer: PCT/EP1999/004674
(87) Internationale Veröffentlichungsnummer: WO 2000/005187

(56) Entgegenhaltungen:
- EP-A- 0 699 666
- EP-A- 0 758 644
- BAUMGARTH M ET AL: "(2-Methyl-5(methylsulfonyl)benzoyl) guanidine Na+/H+ antiporter inhibitors" JOURNAL OF MEDICINAL CHEMISTRY, Bd. 40, Nr. 13, 20. Juni 1997 (1997-06-20), Seiten 2017-34, XP002121770
- GILMAN H ET AL: "Some interconversion reactions of organolithium compounds" JOURNAL OF THE AMERICAN CHEMICAL SOCIETY, Bd. 62, Nr. 9, 7. September 1940 (1940-09-07), Seiten 2327-35, XP002121771 in der Anmeldung erwähnt
- GILMAN H ET AL: "Secondary and tertiary alkyllithium compounds and some interconversion reactions with them" JOURNAL OF THE AMERICAN CHEMICAL SOCIETY, Bd. 63, Nr. 9, 5. September 1941 (1941-09-05), Seiten 2479-82, XP002121772

## Beschreibung

Die Erfindung betrifft ein Verfahren zur Herstellung von ortho-alkylierten Benzoesäurederivaten der Formel I 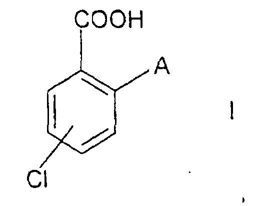 worin
A einen Alkylrest mit 1 bis 4 C-Atomen
bedeutet,
dadurch gekennzeichnet, daß man ein Arylbromid der Formel II 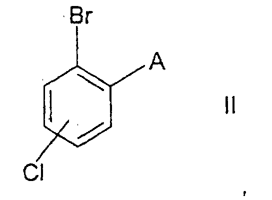 worin A die in Formel I angegebene Bedeutung hat,
mit einem sekundären oder tertiären Lithiumorganyl und CO₂ umsetzt.

Ortho-alkylierte Benzoesäurederivate der Formel I sind wichtige Zwischenstufen in der großtechnischen organischen Synthese, z.B. bei der Herstellung von Feinchemikalien, Farbstoffen und Pflanzenschutzmitteln. Sie sind weiterhin wichtige Zwischenstufen bei der Herstellung von Arzneimitteln, insbesondere bei der Herstellung von Inhibitoren des zellulären Na⁺/H⁺-Antiporters, die aus EP 0 699 666 A1 oder EP 0 758 644 bekannt sind. Insbesondere 4-Chlor-2-methyl-benzoesäure ist ein Zwischenprodukt bei der Synthese von N-Diaminomethylen-2-methyl-4-(1-pyrrolyl)-5-methylsulfonylbenzamid, bekannt aus EP 0 699 666 A1 oder N-Diaminomethylen-2-methyl-4,5-di-(methylsulfonyl)-benzamid, bekannt aus EP 0 758 644.

Aus der klassischen organischen Synthese (s. dazu Standardwerke der organischen Synthese wie Houben-Weyl, Methoden der organischen Chemie, Georg-Thieme-Verlag, Stuttgart oder Beyer, Walter, Lehrbuch der organischen Synthese, S. Hirzel Verlag, Stuttgart) ist die Herstellung von ortho-alkylierten Benzoesäurederivaten aus Anilinderivaten durch Diazotierung, Sandmeyer-Reaktion und anschließender Nitrilverseifung oder durch ortho-Metallierung und anschließender Alkylierung von Benzoesäurederivaten bekannt. Aus ökonomischen und ökologischen Gründen sind diese mehrstufigen Synthesesequenzen für eine großtechnische Anwendung unpraktikabel.

In H. Gilman et al., J. Am. Chem. Soc. 1940, 62, 2327f. ist die Synthese von Benzoesäurederivaten durch Lithiierung der entsprechenden Arylbromide und anschließender Carboxylierung beschrieben. Die Herstellung von 2-Methyl-benzoesäure durch Umsetzung von o-Bromtoluol mit n-Butyllithium und anschließender Carboxylierung mit festem CO₂ gelingt mit einer Ausbeute von 83.8%. Die Umsetzung von 4-Chlorbrombenzol mit n-Butyllithium und CO₂ ergibt 4-Chlor-benzoesäure mit 90%iger Ausbeute.

Eine Herstellung der ortho-alkylierten Verbindungen der Formel I, insbesondere von 4-Chlor-2-methyl-benzoesäure, ist mit Hilfe der zuvor beschriebenen Synthese nicht möglich. Unter den in H. Gilman et al., J. Am. Chem. Soc. **1940,** 62, 2327f. beschriebenen Reaktionsbedingungen und Verwendung der üblichen Lithiierungsreagenzien n-Butyllithium, n-Hexyllithium, Phenyllithium oder Methyllithium findet die gewünschte Reaktion nicht oder nur mit geringer Ausbeute statt.

Diese Befunde werden femer durch die in US 3,910,947 beschriebene Synthese von 4-Chlor-2-methyl-benzoesäure unterstützt. Durch Diazotierung von 2-Methyl-4-chlor-anilin und Abfangen des Diazoniumsalzes mit Kl wird zunächst das sehr reaktive 2-lod-4-chlor-toluol synthetisiert, das sofort durch Reaktion mit n-Butyllithium und CO₂ in 4-Chlor-2-methyl-benzoesäure umgewandelt wird. Die Wahl des sehr reaktiven Aryliodids im Vergleich zu dem billigeren und leichter zugänglichen Bromderivaten bestätigt, daß es bisher nicht möglich war, die Arylbromide in zufriedenstellender Ausbeute zu den gewünschten Benzoesäurederivaten der Formel I umzuwandeln.

Der Erfindung lag daher die Aufgabe zugrunde, ein Verfahren zur Herstellung von ortho-alkylierten Benzoesäurederivaten der Formel I zu entwickeln, das den Einsatz von Arylbromiden ermöglicht.

Überraschenderweise wurde gefunden, daß die Reaktion der Arylbromide der Formel II mit einem sekundären oder tertiären Lithiumorganyl als Metallierungsmittel mit im Vergleich zum Stand der Technik verbesserter oder sehr guter Ausbeute stattfindet.

Es wurde dadurch die Möglichkeit geschaffen, die ortho-alkylierten Benzoesäurederivate der Formel I durch eine auch großtechnisch leicht handhabbare Reaktion als Eintopfsynthese unter milden Bedingungen und mit dem Einsatz der im Vergleich zu den Aryliodiden billigeren Bromderivaten der Formel II herzustellen.

BAUMGARTH M ET AL: ('(2-Methyl-5(methylsulfonyl)benzoyl) guanidine Na+/H+ antiporter inhibitors' JOURNAL OF MEDICAL CHEMITRY, Bd. 40, Nr. 13, 20. Juni 1997 (1997-06-20), Seiten 2017-34, XP002121770) offenbaren ein Verfahren zur Herstellung von ortho-alkylierten Benzoesäurederivaten und ein Verfahren zur Herstellung von Alkylbenzoylguanidin-Derivativen, die jedoch nicht die erfindungsgemäße Umsetzung mit einem sekundären oder tertiären Lithium organyl beinhalten.

Gegenstand der Erfindung ist daher ein Verfahren zur Herstellung von ortho-alkylierten Benzoesäurederivaten der Formel I 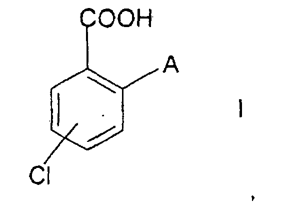 dadurch gekennzeichnet, daß man ein Arylbromid der Formel II 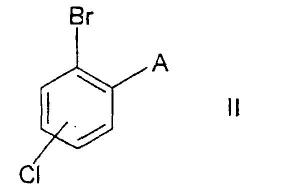 mit einem sekundären oder tertiären Lithiumorganyl und CO₂ umsetzt.

Gegenstand der Erfindung ist ferner ein Verfahren zur Herstellung von ortho-alkylierten Benzoesäuren der Formel I, dadurch gekennzeichnet, daß man ein sekundäres Lithiumorganyl, ausgewählt aus der Gruppe sec.-Butyllithium, iso-Propyllithium, sec.-Amyllithium, 4-Heptyllithium, Cyclopropyllithium oder Cyclohexyllithium oder ein tertiäres Lithiumorganyl, ausgewählt aus der Gruppe tert.-Butyllithium, tert.-Amyllithium, Triethylmethyllithium, 1-Methyl-cyclopentyllithium oder Adamantyllithium einsetzt.

Gegenstand der Erfindung ist ferner ein Verfahren zur Herstellung von ortho-alkylierten Benzoesäurederivaten der Formel I, dadurch gekennzeichnet, daß man die Reaktion bei Temperaturen zwischen -100° und +50°C durchführt und das Reaktionsprodukt durch Zugabe einer Säure fällt.

Gegenstand der Erfindung ist weiterhin ein Verfahren zur Herstellung von ortho-alkylierten Benzoesäurederivaten der Formel I, dadurch gekennzeichnet, daß man die Reaktion in einem inerten Lösungsmittel durchführt, ausgewählt aus der Gruppe Diethylether, Methyl-tert.-butylether, Tetrahydrofuran, Dioxan, Toluol, Hexan, Petrolether oder Mischungen davon.

Gegenstand der Erfindung ist ferner ein Verfahren zur Herstellung von ortho-alkylierten Benzoesäurederivaten der Formel I, dadurch gekennzeichnet, daß man das Arylbromid der Formel II in einem inerten Lösungsmittel vorlegt, das sekundäre oder tertiäre Lithiumorganyl zugibt, dieses Reaktionsgemisch in CO₂-gesättigtes Lösungsmittel tropft und nochmals mit CO₂ sättigt.

Gegenstand der Erfindung ist bevorzugt ein Verfahren zur Herstellung von 4-Chlor-2-methyl-benzoesäure, dadurch gekennzeichnet, daß man 2-Brom-5-chlor-toluol mit einem sekundären oder tertiären Lithiumorganyl und CO₂ umsetzt.

Gegenstand der Erfindung ist besonders bevorzugt ein Verfahren zur Herstellung von 4-Chlor-2-methyl-benzoesäure, dadurch gekennzeichnet, daß man
a) 2-Brom-5-chlor-toluol mit sec.-Butyllithium und CO₂ umsetzt,
b) die Reaktion bei Temperaturen zwischen -100° und +50°C durchführt und 4-Chlor-2-methyl-benzoesäure durch Zugabe einer Säure fällt,
c) die Reaktion in einem inerten Lösungsmittel durchführt, ausgewählt aus der Gruppe Diethylether, Methyl-tert.-butyl-ether, Tetrahydrofuran, Dioxan, Toluol, Hexan, Petrolether oder Mischungen davon und
d) das 2-Brom-5-chlor-toluol in einem inerten Lösungsmittel vorlegt, das sec.-Butyllithium zugibt, dieses Reaktionsgemisch in CO₂-gesättigtes Lösungsmittel tropft und nochmals mit CO₂ sättigt.

Gegenstand der Erfindung ist weiterhin die Verwendung des obengenannten Verfahrens zur Herstellung von 4-chlor-2-methyl benzoesäure, bei der Synthese von N-Diaminomethylen-2-methyl-4-(1-pyrrolyl)-5-methylsulfonyl-benzamid.

Gegenstand der Erfindung ist auch ein Verfahren zur Herstellung von N-Diaminomethylen-2-methyl-4-(1-pyrrolyl)-5-methylsulfonylbenzamid
dadurch gekennzeichnet, daß man
in Stufe a) 2-Brom-5-chlor-toluol mit einem sekundären oder tertiären Lithiumorganyl, besonders bevorzugt sec.-Butyllithium, und CO₂ zu 4-Chlor-2-methyl-benzoesäure umsetzt,
in Stufe b) 4-Chlor-2-methyl-benzoesäure mit Chlorsulfonsäure, Natriumsulfit und Methyliodid zu 2-Methyl-4-chlor-5-methylsulfonylbenzoesäure umsetzt,
in Stufe c) 2-Methyl-4-chlor-5-methylsulfonyl-benzoesäure mit Benzylamin zu 4-Benzylamino-5-methylsulfonyl-2-methyl-benzoesäure umsetzt,
in Stufe d) 4-Benzylamino-5-methylsulfonyl-2-methyl-benzoesäure mit einem Alkohol zu dem entsprechenden Ester von 4-Benzylamino-5-methylsulfonyl-2-methyl-benzoesäure verestert,
in Stufe e) der Ester aus Stufe d) zum entsprechenden 4-Amino-5-methylsulfonyl-2-methyl-benzoesäureester reduziert,
in Stufe f) 4-Amino-5-methylsulfonyl-2-methyl-benzoesäureester mit Dimethoxytetrahydrofuran zu 2-Methyl-4-(1-pyrrolyl)-5-methylsulfonylbenzoesäureester umsetzt und
in Stufe g) 2-Methyl-4-(1-pyrrolyl)-5-methylsulfonyl-benzoesäureester mit Guanidin zu N-Diaminomethylen-2-methyl-4-(1-pyrrolyl)-5-methylsulfonylbenzamid umsetzt.

Bevorzugt wird in Stufe d) ein aliphatischer Alkohol mit 1 bis 6 C-Atomen eingesetzt, wie z.B. Methanol, Ethanol, Propanol, Butanol, Pentanol oder Hexanol. Besonders bevorzugt verwendet wird Methanol.

Gegenstand der Erfindung ist weiterhin die Verwendung des obengenannten Verfahrens zur Herstellung von 4-Chlor-2-bei der Synthese von N-Diaminomethylen-2-methyl-4,5-di-(methylsulfonyl)-benzamid.

Gegenstand der Erfindung ist auch ein Verfahren zur Herstellung von N-Diaminomethylen-2-methyl-4,5-di-(methylsulfonyl)-benzamid dadurch gekennzeichnet, daß man
in Stufe a) 2-Brom-5-chlor-toluol mit einem sekundären oder tertiären Lithiumorganyl, besonders bevorzugt sec.-Butyllithium, und CO₂ zu 4-Chlor-2-methyl-benzoesäure umsetzt,
in Stufe b) 4-Chlor-2-methyl-benzoesäure mit Chlorsulfonsäure, Natriumsulfit und Methyliodid zu 2-Methyl-4-chlor-5-methylsulfonylbenzoesäure umsetzt,
in Stufe c) 2-Methyl-4-chlor-5-methylsulfonyl-benzoesäure mit Natriummethylthiolat umsetzt und anschließend mit einem Oxidationsmittel zu 2-Methyl-4,5-di-(methylsulfonyl)-benzoesäure oxidiert,
in Stufe d) 2-Methyl-4,5-di-(methylsulfonyl)-benzoesäure mit Thionylchlorid zu 2-Methyl-4,5-di-(methylsulfonyl)-benzoesäurechlorid umsetzt und
in Stufe e) 2-Methyl-4,5-di-(methylsulfonyl)-benzoesäurechlorid mit Guanidiniumchlorid zu N-Diaminomethylen-2-methyl-4,5-di-(methylsulfonyl)-benzamid umsetzt.

Bevorzugte Oxidationsmittel der Stufe c) sind H₂O₂, O₂ oder Natriumperborat. Ganz besonders bevorzugt wird Natriumperborat eingesetzt.

Die verwendeten Abkürzungen haben die folgenden Bedeutungen:

| | |
|---|---|
| n-Bu | n-Butyl |
| Et | Ethyl |
| Me | Methyl |
| MTB | Methyl-tert.-butyl |
| THF | Tetrahydrofuran |
| h | Stunde |

In den vorstehenden Formeln ist A Alkyl und hat 1 bis 4, vorzugsweise 1, 2 oder 3 C-Atome. Alkyl bedeutet vorzugsweise Methyl, weiterhin Ethyl, Propyl, Isopropyl, Butyl, Isobutyl, sec.-Butyl oder tert.-Butyl. Besonders bevorzugt ist Methyl.

In den vorstehenden Formeln steht der Cl-Substituent vorzugsweise in der 3-, 4-, 5- oder 6-Stellung, besonders bevorzugt in der 4-Stellung zur Position der Carboxylgruppe der Formel I.

Die Arylbromide der Formel II sind kommerziell erhältlich oder können nach an sich bekannten Methoden wie z.B. in Houben-Weyl, Methoden der Organ. Chemie beschrieben, hergestellt werden.

CO₂ wird fest oder gasförmig eingesetzt.

Gegenstand der Erfindung ist auch ein Verfahren, wie beschrieben,
dadurch gekennzeichnet, daß man sekundäre Lithiumorganyle ausgewählt aus der Gruppe sec.-Butyllithium, iso-Propyllithium, sec.-Amyllithium, 4-Heptyllithium, Cyclopropyllithium oder Cyclohexyllithium oder ein tertiäres Lithiumorganyl, ausgewählt aus der Gruppe tert.-Butyllithium, tert.-Amyllithium, Triethylmethyllithium, 1-Methyl-cyclopentyllithium oder Adamantyllithium einsetzt.

Bevorzugt ist der Einsatz von sekundären Lithiumorganylen, ausgewählt aus der Gruppe sec.-Butyllithium, iso-Propyllithium, sec.-Amyllithium, 4-Heptyllithium, Cyclopropyllithium oder Cyclohexyllithium; besonders bevorzugt die Verwendung von sec.-Butyllithium.

Die vorab angeführten sekundären oder tertiären Lithiumorganyle sind kommerziell erhältlich oder können nach an sich bekannten Methoden wie z.B. in Houben-Weyl, Methoden der Organ. Chemie beschrieben, hergestellt werden.

Gegenstand der Erfindung ist auch ein Verfahren, wie beschrieben,
. dadurch gekennzeichnet, daß man die Reaktion bei Temperaturen zwischen -100° und +50°C durchführt. Bevorzugt ist der Temperaturbereich zwischen -50° und +40°, besonders bevorzugt ist der Temperaturbereich zwischen -20° und +5°C, ganz besonders bevorzugt ist der Temperaturbereich zwischen -15° und 0°C.

Gegenstand der Erfindung ist auch ein Verfahren, wie beschrieben,
dadurch gekennzeichnet, daß man das Reaktionsprodukt nach üblicher Aufarbeitung des Reaktionsgemisches sauer fällt. Es bedeutet übliche Aufarbeitung: Man gibt NaOH (10%) zum Reaktionsgemisch, trennt die Phasen, wäscht die organische Phase mit NaOH (10%), extrahiert die wäßrigen Phasen mit dem inerten Lösungsmittel und trennt ab. Die Säure wird ausgewählt aus einer Gruppe von Säuren, zu der organische Säuren, bevorzugt Ameisensäure, Essigsäure oder Propionsäure oder auch anorganische Säuren, bevorzugt Schwefelsäure, Salpetersäure, Halogenwasserstoffsäuren wie Chlorwasserstoffsäure oder Bromwasserstoffsäure oder Phosphorsäuren wie Orthophosphorsäure gehören. Besonders bevorzugt eingesetzt wird Chlorwasserstoffsäure.

Gegenstand der Erfindung ist auch ein Verfahren, wie beschrieben,
dadurch gekennzeichnet, daß man die Reaktion in einem inerten Lösungsmittel durchführt, ausgewählt aus der Gruppe Diethylether, MTB-Ether, THF, Dioxan, Toluol, Hexan, Petrolether oder Mischungen davon. Besonders bevorzugt ist Methyl-tert.-butyl-ether.

Gegenstand der Erfindung ist auch ein Verfahren, wie beschrieben,
dadurch gekennzeichnet, daß man das Arylbromid der Formel II in einem inerten Lösungsmittel, ausgewählt aus der Gruppe Diethylether, MTB-Ether, THF, Dioxan, Toluol, Hexan, Petrolether oder Mischungen davon, besonders bevorzugt MTB-Ether, vorlegt, das sekundäre oder tertiäre Lithiumorganyl zugibt, dieses Reaktionsgemisch in ein bevorzugtes Volumen von CO₂-gesättigtem Lösungsmittel tropft und nochmals mit CO₂ sättigt.

Entsprechend dem erfindungsgemäßen Verfahren liegen die Ausbeuten an ortho-alkylierten Benzoesäurederivaten der Formel I bei Verwendung von sekundären oder tertiären Lithiumorganylen in der Regel zwischen 30% und 90%, bei Verwendung von sekundären Lithiumorganylen zwischen 50% und 90%, insbesondere bei Verwendung von sec.-Butyllithium zwischen 70% und 90%. Aufwendige Reinigungsschritte durch z.B. mehrfache Umkristallisation können entfallen. Vor- und nachstehend sind alle Temperaturen in °C angegeben. Die Gehaltsbestimmungen erfolgten z.B. nach Trocknung des Kristallisats bei 55°.

Gegenstand der Erfindung ist auch die Verwendung von 4-Chlor-2-methylbenzoesäure, hergestellt nach dem zuvor beschriebenen Verfahren, als Zwischenprodukt bei der Synthese von N-Diaminomethylen-2-methyl-4-(1-pyrrolyl)-5-methylsulfonyl-benzamid, bekannt aus EP 0 699 666 A1. Weitere Zwischenprodukte dieser Synthesesequenz von N-Diaminomethylen-2-methyl-4-(1-pyrrolyl)-5-methylsulfonyl-benzamid, ausgehend von dem Zwischenprodukt 4-Chlor-2-methyl-benzoesäure, sind 2-Methyl-4-chlor-5-methylsulfonyl-benzoesäure, 2-Methyl-4-chlor-5-methylsulfonyl-benzoesäuremethylester und 2-Methyl-4-(1-pyrrolyl)-5-methylsulfonyl-benzoesäuremethylester.

Demgemäß ist auch Gegenstand der Erfindung ein Verfahren zur Herstellung von N-Diaminomethylen-2-methyl-4-(1-pyrrolyl)-5-methylsulfonylbenzamin, dadurch gekennzeichnet, daß man in der ersten Stufe a) erfindungsgemäß 2-Brom-5-chlor-toluol mit einem sekundären oder tertiären Lithiumorganyl, besonders bevorzugt sec.-Butyllithium, und CO₂ zum ersten Zwischenprodukt 4-Chlor-2-methyl-benzoesäure umsetzt, in Stufe b) eine Methylsulfonylgruppe eingeführt wird, indem 4-Chlor-2-methyl-benzoesäure mit Chlorsulfonsäure, Natriumsulfit und Methyliodid zum entsprechenden Zwischenprodukt 2-Methyl-4-chlor-5-methylsulfonylbenzoesäure umgesetzt wird,
in Stufe c) der Chlorsubstituent in eine Benzylaminogruppe umgewandelt wird, indem man 2-Methyl-4-chlor-5-methylsulfonyl-benzoesäure mit Benzylamin zum entsprechenden Zwischenprodukt 4-Benzylamino-5-methylsulfonyl-2 -methyl-benzoesäure umsetzt,
in Stufe d) die freie Säure aus Stufe c) mit einem Alkohol verestert, insbesondere Methanol, und das entsprechende Zwischenprodukt 4-Benzylamino-5-methylsulfonyl-2-methyl-benzoesäureester erhalten wird,
in Stufe e) die Benzylschutzgruppe durch Reduktion unter Erhalt des entsprechenden Zwischenprodukts 4-Amino-5-methylsulfonyl-2-methylbenzoesäureester abgespalten wird,
in Stufe f) die Pyrrolgruppe durch Umsetzung von 4-Amino-5-methylsulfonyl-2-methyl-benzoesäureester mit Dimethoxytetrahydrofuran eingeführt und entsprechend das Zwischenprodukt 2-Methyl-4-(1-pyrrolyl)-5-methylsulfonylbenzoesäureester erhalten wird und abschließend, wie in EP 0 699 666 beschrieben (S. 8 Zeile 19 bis S. 10, Zeile 1),
in Stufe g) durch Reaktion von 2-Methyl-4-(1-pyrrolyl)-5-methylsulfonylbenzoesäuremethylester mit Guanidin unter Erhalt des Endprodukts N-Diaminomethylen-2-methyl-4-(1-pyrrolyl)-5-methylsulfonylbenzamid die Guanidinogruppe eingeführt wird.

Gegenstand der Erfindung ist auch die Verwendung von 4-Chlor-2-methylbenzoesäure, hergestellt nach dem zuvor beschriebenen Verfahren, als Zwischenprodukt bei der Synthese von N-Diaminomethylen-2-methyl-4,5-di-(methylsulfonyl)-benzamid, bekannt aus EP 0 758 644 A1. Weitere Zwischenprodukte dieser Synthesesequenz von N-Diaminomethylen-2-methyl-4,5-di-(methylsulfonyl)-benzamid, ausgehend von dem Zwischenprodukt 4-Chlor-2-methyl-benzoesäure, sind 2-Methyl-4-chlor-5-methylsulfonyl-benzoesäure, 2-Methyl-4,5-di-(methylsulfonyl)-benzoesäure und 2-Methyl-4,5-di-(methylsulfonyl)-benzoesäurechlorid.

Demgemäß ist auch Gegenstand der Erfindung ein Verfahren zur Herstellung von N-Diaminomethylen-2-methyl-4,5-di-(methylsulfonyl)-benzamin, dadurch gekennzeichnet, daß man in der ersten Stufe a) erfindungsgemäß 2-Brom-5-chlor-toluol mit einem sekundären oder tertiären Lithiumorganyl, besonders bevorzugt sec.-Butyllithium, und CO₂ zum ersten Zwischenprodukt 4-Chlor-2-methyl-benzoesäure umsetzt, in Stufe b) eine Methylsulfonylgruppe eingeführt wird, indem 4-Chlor-2-methyl-benzoesäure mit Chlorsulfonsäure, Natriumsulfit und Methyliodid zum entsprechenden Zwischenprodukt 2-Methyl-4-chlor-5-methylsulfonylbenzoesäure umgesetzt wird,
in Stufe c) der Chlorsubstituent in eine zweite Methylsulfonylgruppe umgewandelt wird, indem man 2-Methyl-4-chlor-5-methylsulfonylbenzoesäure mit Natriummethylthiolat umsetzt und anschließend den Thioether mit einem Oxidationsmittel, insbesondere Natriumperborat, zum entsprechenden Zwischenprodukt 2-Methyl-4,5-di-(methylsulfonyl)-benzoesäure oxidiert,
in Stufe d) die freie Säure aus Stufe c) mit Thionylchlorid in das Säurechlorid 2-Methyl-4,5-di-(methylsulfonyl)-benzoesäurechlorid als Zwischenstufe umwandelt und
in Stufe e) abschließend, wie in EP 0 758 644 beschrieben (S. 9, Zeilen 10-20), durch Reaktion von 2-Methyl-4,5-di-(methylsulfonyl)-benzoesäurechlorid mit Guanidiniumchlorid unter Erhalt des Endprodukts N-Diaminomethylen-2-methyl-4,5-di-(methylsulfonyl)benzamid die Guanidinogruppe eingeführt wird.

Bei den nachfolgenden Beispielen und auch bei den vorherigen Ausführungen wird die Temperatur in °C angegeben. Der pH-Wert entspricht dem dekadischen Logarithmus der H⁺-Ionenkonzentration.

### Beispiel 1:

Zu einer Lösung von 104.8 g 2-Brom-5-chlor-toluol in 500 ml MTB-Ether werden bei einer Temperatur von -18° 728 ml sec.-Butyllithium innerhalb von 30 min. gegeben. Die Suspension wird anschließend innerhalb von 20 min. in 750 ml CO₂-gesättigten MTB-Ether getropft und nochmals 15 min. mit gasförmigem CO₂ gesättigt. Nach einer Reaktionszeit von 1 h bei Temperaturen zwischen -15° und -5° werden 500 ml NaOH (10%) zugegeben und die Phasen getrennt. Die organische Phase wird mit 250 ml NaOH (10%) gewaschen. Die vereinigten wäßrigen Phasen werden mit 250 ml MTB-Ether extrahiert, mit 250.4 g HCl (37%) auf pH 1-2 eingestellt . und 1 h bei 5° im Eis-Wasser-Bad gekühlt. Das Kristallisat wird zwei Mal mit 50 ml kaltem Wasser gewaschen und im Vakuum bei 55° getrocknet. Die Ausbeute an 4-Chlor-2-methyl-benzoesäure beträgt 90%.

### Beispiel 2:

Im Vergleich wird analog zu Beispiel 1 2-Brom-5-chlor-toluol mit den in der Tabelle I ausgeführten Lithiierungsreagenzien in den in Tabelle I angegebenen Lösungsmitteln umgesetzt. Die Ausbeute an 4-Chlor-2-methyl-benzoesäure ist zwischen 0 und 30%.

**Tabelle I:**

| Lithiierungsreagenz | Lösungsmittel | Ausbeute [%] |
|---|---|---|
| MeLi | MTB-Ether | 0 |
| n-BuLi | MTB-Ether | 0 |
| n-BuLi | THF | 30 |

### Beispiel 3:

Im Vergleich wird analog zu H. Gilman et al., J. Am. Chem. Soc. 1940, 62, 2327f. 2-Brom-5-chlor-toluol mit n-BuLi in siedendem Diethylether umgesetzt. Die Ausbeute an 4-Chlor-2-methyl-benzoesäure ist 20%.

### Beispiel 4:

Analog zu Beispiel 1 werden 20,5 g 2-Brom-5-chlor-toluol, gelöst in Methyltert.-butyl-ether, mit 143 ml tert.-Butyllithium und CO₂ umgesetzt. Die Ausbeute an 4-Chlor-2-methyl-benzoesäure beträgt 32%.

### Beispiel 5:

Synthese von N-Diaminomethylen-2-methyl-4-(1-pyrrolyl)-5-methylsulfonylbenzamid:
a) Zu einer Lösung von 104.8 g 2-Brom-5-chlor-toluol in 500 ml MTB-Ether werden bei einer Temperatur von -18° 728 ml sec.-Butyllithium innerhalb von 30 min. gegeben. Die Suspension wird anschließend innerhalb von 20 min. in 750 ml CO₂-gesättigten MTB-Ether getropft und nochmals 15 min. mit gasförmigem CO₂ gesättigt. Nach einer Reaktionszeit von 1 h bei Temperaturen zwischen -15° und -5° werden 500 ml NaOH (10%) zugegeben und die Phasen getrennt. Die organische Phase wird mit 250 ml NaOH (10%) gewaschen. Die vereinigten wäßrigen Phasen werden mit 250 ml MTB-Ether extrahiert, mit 250.4 g HCl (37%) auf pH 1-2 eingestellt und 1 h bei 5° im Eis-Wasser-Bad gekühlt. Das Kristallisat wird zwei Mal mit 50 ml kaltem Wasser gewaschen und im Vakuum bei 55° getrocknet.
   Die Ausbeute an 4-Chlor-2-methyl-benzoesäure beträgt 90%.
b) Unter Eiskühlung werden 722 g 4-Chlor-2-methyl-benzoesäure bei 15° in 2,4 l Chlorsulfonsäure gelöst. Nach Erwärmen auf 110-115° wird die Lösung in Eiswasser (20 l) getropft und nachgerührt. Der Niederschlag wird von der Mutterlauge getrennt, getrocknet und anschließend in eine Suspension von 1333 g Natriumsulfit in 3 l Wasser gegeben. Gleichzeitig wird der pH-Wert durch Zugabe von Natronlauge auf pH 9 gehalten. Nach vier Stunden Rühren bei Raumtemperatur wird der pH-Wert der Suspension mit Salzsäure auf pH 1 gestellt. Der Niederschlag wird von der Mutterlauge getrennt und in 3 l Methanol und 2 l Wasser suspendiert. Zu dieser Suspension werden 1,3 l Methyliodid zugegeben und der pH mit Natronlauge auf pH 9 gestellt und auf 40° erhitzt. Nach Abdestillieren von Methanol und überschüssigem Methyliodid wird mit Wasser verdünnt und mit Essigsäureethylester extrahiert. Essigsäureethylester wird zum Teil abdestilliert, die verbleibende Lösung auf pH 1 eingestellt und der daraufhin entstehende Feststoff von der Mutterlauge getrennt und getrocknet.
   Man erhält 2-Methyl-4-chlor-5-methylsulfonyl-benzoesäure in einer Ausbeute von 67%.
c) Eine Lösung von 684 g 2-Methyl-4-chlor-5-methylsulfonylbenzoesäure und 884 g Benzylamin in 4 l N-Methylpyrrolidin wird 8 h bei 160° gerührt. Die Lösung wird anschließend auf Wasser gegossen, der pH-Wert mit Natonlauge auf pH 12 eingestellt und mit Essigsäureethylester extrahiert. Die wäßrige Phase wird mit Salzsäure auf pH 1 eingestellt. Der entstehende Niederschlag wird abfiltriert und über Nacht getrocknet.
   Man erhält 4-Benzylamino-5-methylsulfonyl-2-methyl-benzoesäure in einer Ausbeute von 87%.
d) Eine Suspension von 767 g 4-Benzylamino-5-methytsulfonyl-2-methyl-benzoesäure in 12 l Methanol wird auf Rückfluß erwärmt. Gleichzeitig wird HCl-Gas eingeleitet. Nach vollständigem Umsatz wird die klare Lösung auf Eis gegossen und der entstehende Niederschlag abfiltriert und getrocknet.
   Man erhält 4-Benzylamino-5-methylsulfonyl-2-methyl-benzoesäuremethylester in einer Ausbeute von 96%.
e) Zu einer Lösung von 683 g 4-Benzylamino-5-methylsulfonyl-2-methylbenzoesäure-methylester in 6,8 l Methanol werden 68 g Pd-C (5%) zugegeben und 48,4 l Wasserstoff zugeführt. Nach 4 h wird mit 5 l Methylenchlorid verdünnt, der Katalysator abfiltriert und das Lösungsmittel zum Teil abdestilliert. Der entstehende Niederschlag wird von der eingeengten Mutterlauge abfiltriert und getrocknet.
   Man erhält 4-Amino-5-methylsulfonyl-2-methyl-benzoesäure-methylester in einer Ausbeute von 98%.
f) Zu einer Lösung von 385,9 g 4-Amino-5-methylsulfonyl-2-methylbenzoesäure-methylester in 6 l 1,4-Dioxan werden 260 ml Dimethoxytetrahydrofuran und 23,8 g 4-Chlorpyridiniumchlorid zugegeben. Die Lösung wird unter Rückfluß bis zum vollständigen Umsatz erhitzt und das Lösungsmittel abdestilliert. Der Rückstand wird in Essigsäureethylester aufgenommen und mit Wasser gewaschen und über Na₂SO₄ getrocknet. Anschließend wird mit 15 g Aktivkohle unter Rückfluß entfärbt, von der Aktivkohle abfiltriert und der Essigsäureethylester abdestilliert. Der entstehende Rückstand wird aus Methanol umkristallisiert.
   Man erhält 2-Methyl-4-(1-pyrrolyl)-5-methylsulfonylbenzoesäuremethylester in einer Ausbeute von 89%.
g) Man rührt eine Lösung von 694 g Guanidin und 310 g 2-Methyl-4-(1-pyrrolyl)-5-methylsulfonyl-benzoesäuremethylester in 3 l Methanol 3 h bei 50°. Anschließend versetzt man die Reaktionsmischung mit Wasser, filtriert das dabei entstehende Rohprodukt ab und kristallisiert aus Methanol um.
   Man erhält N-Diaminomethylen-2-methyl-4-(1-pyrrolyl)-5-methylsulfonylbenzamid in einer Ausbeute von 66%.

### Beispiel 6:

Synthese von N-Diaminomethylen-2-methyl-4,5-di-(methylsulfonyl)-benzamid:
a) Zu einer Lösung von 104.8 g 2-Brom-5-chlor-toluol in 500 ml MTB-Ether werden bei einer Temperatur von -18° 728 ml sec.-Butyllithium innerhalb von 30 min. gegeben. Die Suspension wird anschließend innerhalb von 20 min. in 750 ml CO₂-gesättigten MTB-Ether getropft und nochmals 15 min. mit gasförmigem CO₂ gesättigt. Nach einer Reaktionszeit von 1 h bei Temperaturen zwischen -15° und -5° werden 500 ml NaOH (10%) zugegeben und die Phasen getrennt. Die organische Phase wird mit 250 ml NaOH (10%) gewaschen. Die vereinigten wäßrigen Phasen werden mit 250 ml MTB-Ether extrahiert, mit 250.4 g HCl (37%) auf pH 1-2 eingestellt und 1 h bei 5° im Eis-Wasser-Bad gekühlt. Das Kristallisat wird zwei Mal mit 50 ml kaltem Wasser gewaschen und im Vakuum bei 55° getrocknet.
   Die Ausbeute an 4-Chlor-2-methyl-benzoesäure beträgt 90%.
b) Unter Eiskühlung werden 722 g 4-Chlor-2-methyl-benzoesäure bei 15° in 2,4 l Chlorsulfonsäure gelöst. Nach Erwärmen auf 110-115° wird die Lösung in Eiswasser (20 l) getropft und nachgerührt. Der Niederschlag wird von der Mutterlauge getrennt, getrocknet und anschließend in eine Suspension von 1333 g Natriumsulfit in 3 l Wasser gegeben. Gleichzeitig wird der pH-Wert durch Zugabe von Natronlauge auf pH 9 gehalten. Nach vier Stunden Rühren bei Raumtemperatur wird der pH-Wert der Suspension mit Salzsäure auf pH 1 gestellt. Der Niederschlag wird von der Mutterlauge getrennt und in 3 l Methanol und 2 l Wasser suspendiert. Zu dieser Suspension werden 1,3 l Methyliodid zugegeben und der pH mit Natronlauge auf pH 9 gestellt und auf 40° erhitzt. Nach Abdestillieren von Methanol und überschüssigem Methyliodid wird mit Wasser verdünnt und mit Essigsäureethylester extrahiert. Essigsäureethylester wird zum Teil abdestilliert, die verbleibende Lösung auf pH 1 eingestellt und der daraufhin entstehende Feststoff von der Mutterlauge getrennt und getrocknet.
   Man erhält 2-Methyl-4-chlor-5-methylsulfonyl-benzoesäure in einer Ausbeute von 67%.
c) Zu einer Lösung von 600 g 2-Methyl-4-chlor-5-methylsulfonylbenzoesäure in 4 l DMF werden 360 g Natriummethylthiolat gegeben und bis zur vollständigen Umsetzung bei 130° gerührt. Danach wird auf Eiswasser gegossen und der pH-Wert mit Salzsäure auf pH 1 gestellt. Der entsehende Niederschlag wird abfiltriert und getrocknet. Man erhält 5-Methylsulfonyl-2-methyl-4-methylsulfanyl-benzoesäure in einer Ausbeute von 86%.
   Anschließend werden 73 g 5-Methylsulfonyl-2-methyl-4-methylsulfanylbenzoesäure in 1 l Eisessig gelöst und 180 g Natriumperborat zugegeben. Die Reaktionsmischung wird 1 h auf 65° Innentemperatur erhitzt. Eisessig wird weitgehend abdestilliert und der verbleibende Rückstand mit Essigsäureethylester verrieben. Der entstehende Niederschlag wird abfiltriert und mehrmals mit einer Mischung aus Essigsäureethylester:
   Diethylether 1:1 gewaschen. Zur Entfernung der Borsäure wird der Niederschlag in 1n Salzsäure gerührt, danach abfiltriert und getrocknet. Man erhält 2-Methyl-4,5-di-(methylsulfonyl)-benzoesäure in einer Ausbeute von 50%.
d) Zu 41 g 2-Methy(-4,5-di-(methylsulfonyl)-benzoesäure werden 400 ml Thionylchlorid zugegeben und unter Rückfluß bis zum vollständigen Umsatz erhitzt. Der Überschuß an Thionylchlorid wird abdestilliert und mehrmals mit Toluol kodestilliert.
   Man erhält 2-Methyl-4,5-di-(methylsulfonyl)-benzoesäurechlorid in einer Ausbeute von 98%.
e) Zu einer Lösung von 38,5 g Natrium in 1,3 l Methanol werden 128,4 g Guanidiniumchlorid zugegeben und 30 Minuten bei Raumtemperatur gerührt und filtriert. Nach Entfernen des Lösungsmittels und Waschen mit Toluol wird der Rückstand in 1,3 l Ethylenglykolmonomethylether aufgenommen und zu einer Lösung von 42,8 g 2-Methyl-4,5-di-(methylsulfonyl)-benzoesäurechlorid in 1,7 l Ethylenglykolmonomethylether hinzugefügt. Man rührt 2 h bei Raumtemperatur, verdünnt mit Eiswasser und gibt 1n Salzsäure hinzu. Anschließend wird mit Essigsäureethylester gewaschen und auf einen pH-Wert von pH 9 eingestellt. Nach üblicher Aufarbeitung und Entfernung des Lösungsmittels erhält man nach Umkristallisieren aus Diethylether das N-Diaminomethylen-2-methyl-4,5-di-(methylsulfonyl)-benzamid in einer Ausbeute von 44%.

## Patentansprüche

1. Verfahren zur Herstellung von ortho-alkylierten Benzoesäurederivaten der Formel I 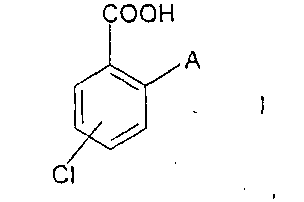 worin
A einen Alkylrest mit 1 bis 4 C-Atomen
bedeutet,
**dadurch gekennzeichnet, daß** man ein Arylbromid der Formel II 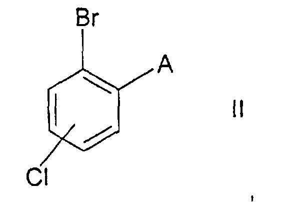 worin A die in Formel I angegebene Bedeutung hat,
mit einem sekundären oder tertiären Lithiumorganyl und CO₂ umsetzt.

2. Verfahren nach Anspruch 1 **dadurch gekennzeichnet, daß** man ein sekundäres Lithiumorganyl, ausgewählt aus der Gruppe sec.-Butyllithium, iso-Propyllithium, sec.-Amyllithium, 4-Heptyllithium, Cyclopropyllithium oder Cyclohexyllithium oder ein tertiäres Lithiumorganyl, ausgewählt aus der Gruppe tert.-Butyllithium, tert.-Amyllithium, Triethylmethyllithium, 1-Methyl-cyclopentyllithium oder Adamantyllithium einsetzt.

3. Verfahren nach Anspruch 1 oder 2 zur Herstellung von 4-Chlor-2-methyl-benzoesäure **dadurch gekennzeichnet, daß** man 2-Brom-5-chlortoluol einsetzt.

4. Verfahren nach Ansprüchen 1 bis 3 **dadurch gekennzeichnet, daß** man die Reaktion bei Temperaturen zwischen -100° und +50°C durchführt und das Reaktionsprodukt durch Zugabe einer Säure fällt.

5. Verfahren nach Ansprüchen 1 bis 4 **dadurch gekennzeichnet, daß** man die Reaktion in einem inerten Lösungsmittel, ausgewählt aus der Gruppe Diethylether, Methyl-tert.-butyl-ether. Tetrahydrofuran, Dioxan, Toluol, Hexan, Petrolether oder Mischungen davon, durchführt.

6. Verfahren nach Ansprüchen 1 bis 5 **dadurch gekennzeichnet, daß** man das Arylbromid der Formel II in einem inerten Lösungsmittel vorlegt, das sekundäre oder tertiäre Lithiumorganyl zugibt, dieses Reaktionsgemisch in CO₂-gesättigtes Lösungsmittel tropft und nochmals mit CO₂ sättigt.

7. Verwendung des Verfahrens nach Anspruch 3 zur Herstellung von 4-Chlor-2-methyl-benzoesäure, bei der Synthese von N-Diaminomethylen-2-methyl-4-(1-pyrrolyl)-5-methylsufonyl-benzamid.

8. Verfahren zur Herstellung von N-Diaminomethylen-2-methyl-4-(1-pyrrolyl)-5-methylsulfonylbenzamid **dadurch gekennzeichnet, daß** man
in Stufe a) 2-Brom-5-chlor-toluol mit einem sekundären oder tertiären Lithiumorganyl, und CO₂ zu 4-Chlor-2-methyl-benzoesäure umsetzt,
in Stufe b) 4-Chlor-2-methyl-benzoesäure mit Chlorsulfonsäure, Natriumsulfit und Methyliodid zu 2-Methyl-4-chlor-5-methylsulfonylbenzoesäure umsetzt,
in Stufe c) 2-Methyl-4-chlor-5-methylsulfonyl-benzoesäure mit Benzylamin zu 4-Benzylamino-5-methylsulfonyl-2-methyl-benzoesäure umsetzt, in Stufe d) 4-Benzylamino-5-methylsulfonyl-2-methyl-benzoesäure mit einem Alkohol zu dem entsprechenden Ester von 4-Benzylamino-5-methylsulfonyl-2-methyl-benzoesäure verestert,
in Stufe e) der Ester aus Stufe d) zum entsprechenden 4-Amino-5-methylsulfonyl-2-methyl-benzoesäureester reduziert,
in Stufe f) 4-Amino-5-methylsulfonyl-2-methyl-benzoesäureester mit Dimethoxytetrahydrofuran zu 2-Methyl-4-(1-pyrrolyl)-5-methylsulfonylbenzoesäureester umsetzt und
in Stufe g) 2-Methyl-4-(1-pyrrolyl)-5-methylsulfonyl-benzoesäureester mit Guanidin zu N-Diaminomethylen-2-methyl-4-(1-pyrrolyl)-5-methylsulfonylbenzamid umsetzt.

9. Verwendung des Verfahrens nach Anspruch 3 zur Herstellung von 4-Chlor-2-methyl-benzoesäure, bei der Synthese von N-Diaminomethylen-2-methyl-4,5-di-(methylsufonyl)-benzamid.

10. Verfahren zur Herstellung von N-Diaminomethylen-2-methyl-4,5-di-(methylsulfonyl)-benzamid **dadurch gekennzeichnet, daß** man
in Stufe a) 2-Brom-5-chlor-toluol mit einem sekundären oder tertiären Lithiumorganyl, und CO₂ zu 4-Chlor-2-methyl-benzoesäure umsetzt,
in Stufe b) 4-Chlor-2-methyl-benzoesäure mit Chlorsulfonsäure, Natriumsulfit und Methyliodid zu 2-Methyl-4-chlor-5 -methylsulfonylbenzoesäure umsetzt,
in Stufe c) 2-Methyl-4-chlor-5-methylsulfonyl-benzoesäure mit Natriummethylthiolat umsetzt und anschließend mit einem Oxidationsmittel zu 2-Methyl-4,5-di-(methylsulfonyl)-benzoesäure oxidiert,
in Stufe d) 2-Methyl-4,5-di-(methylsulfonyl)-benzoesäure mit Thionylchlorid zu 2-Methyl-4,5-di-(methylsulfonyl)-benzoesäurechlorid umsetzt und, in Stufe e) 2-Methyl-4,5-di-(methylsulfonyl)-benzoesäurechlorid mit Guanidiniumchlorid zu N-Diaminomethylen-2-methyl-4,5-di-(methylsulfonyl)-benzamid umsetzt.

## Claims

1. Process for the preparation of ortho-alkylated benzoic acid derivatives of the formula I 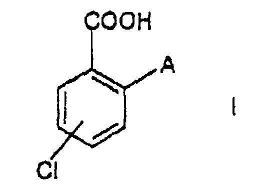 in which
A is an alkyl radical having from 1 to 4 carbon atoms,
**characterised in that** an aryl bromide of the formula II 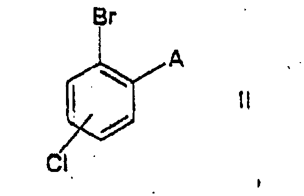 in which A is as defined in formula I,
is reacted with a secondary or tertiary organolithium compound and CO₂.

2. Process according to Claim 1, **characterised in that** a secondary organolithium compound selected from the group consisting of sec-butyllithium, isopropyllithium, sec-amyllithium, 4-heptyllithium, cyclopropyllithium or cyclohexyllithium or a tertiary organolithium compound selected from the group consisting of tert-butyllithium, tert-amyllithium, triethylmethyllithium, 1-methylcyclopentyllithium or adamantyllithium is employed.

3. Process according to Claim 1 or 2 for the preparation of 4-chloro-2-methylbenzoic acid, **characterised in that** 2-bromo-5-chlorotoluene is employed.

4. Process according to Claims 1 to 3, **characterised in that** the reaction is carried out at temperatures between -100° and +50°C and the reaction product is precipitated by addition of an acid.

5. Process according to Claims 1 to 4, **characterised in that** the reaction is carried out in an inert solvent selected from the group consisting of diethyl ether, methyl tert-butyl ether, tetrahydrofuran, dioxane, toluene, hexane, petroleum ether or mixtures thereof.

6. Process according to Claims 1 to 5, **characterised in that** the aryl bromide of the formula II is presented in an inert solvent, the secondary or tertiary organolithium compound is added, this reaction mixture is introduced dropwise into CO₂-saturated solvent and again saturated with CO₂.

7. Use of the process according to Claim 3 for the preparation of 4-chloro-2-methylbenzoic acid in the synthesis of N-diaminomethylene-2-methyl-4-(1-pyrrolyl)-5-methylsulfonylbenzamide.

8. Process for the preparation of N-diaminomethylene-2-methyl-4-(1-pyrrolyl)-5-methylsulfonylbenzamide, **characterised in that**
in step a) 2-bromo-5-chlorotoluene is reacted with a secondary or tertiary organolithium compound and CO₂ to give 4-chloro-2-methylbenzoic acid,
in step b) 4-chloro-2-methylbenzoic acid is reacted with chlorosulfonic acid, sodium sulfite and methyl iodide to give 2-methyl-4-chloro-5-methylsulfonylbenzoic acid;
in step c) 2-methyl-4-chloro-5-methylsulfonylbenzoic acid is reacted with benzylamine to give 4-benzylamino-5-methylsulfonyl-2-methylbenzoic acid, in step d) 4-benzylamino-5-methylsulfonyl-2-methylbenzoic acid is esterified with an alcohol to give the corresponding ester of 4-benzylamino-5-methylsulfonyl-2-methylbenzoic acid,
in step e) the ester from step d) is reduced to the corresponding 4-amino-5-methylsulfonyl-2-methylbenzoic acid ester,
in step f) 4-amino-5-methylsulfonyl-2-methylbenzoic acid ester is reacted with dimethoxytetrahydrofuran to give 2-methyl-4-(1-pyrrolyl)-5-methylsulfonylbenzoic acid ester, and
in step g) 2-methyl-4-(1-pyrrolyl)-5-methylsulfonylbenzoic acid ester is reacted with guanidine to give N-diaminomethylene-2-methyl-4-(1-pyrrolyl)-5-methylsulfonylbenzamide.

9. Use of the process according to Claim 3 for the preparation of 4-chloro-2-methylbenzoic acid in the synthesis of N-diaminomethylene-2-methyl-4,5-di(methylsulfonyl)benzamide.

10. Process for the preparation of N-diaminomethylene-2-methyl-4,5-di(methylsulfonyl)benzamide, **characterised in that**
in step a) 2-bromo-5-chlorotoluene is reacted with a secondary or tertiary organolithium compound and CO₂ to give 4-chloro-2-methylbenzoic acid,
in step b) 4-chloro-2-methylbenzoic acid is reacted with chlorosulfonic acid, sodium sulfite and methyl iodide to give 2-methyl-4-chloro-5-methylsulfonylbenzoic acid,
in step c) 2-methyl-4-chloro-5-methylsulfonylbenzoic acid is reacted with sodium methylthiolate and subsequently with an oxidant to give 2-methyl-4,5-di(methylsulfonyl)benzoic acid,
in step d) 2-methyl-4,5-di(methylsulfonyl)benzoic acid is reacted with thionyl chloride to give 2-methyl-4,5-di(methylsulfonyl)benzoyl chloride, and
in step e) 2-methyl-4,5-di(methylsulfonyl)benzoyl chloride is reacted with guanidinium chloride to give N-diaminomethylene-2-methyl-4,5-di(methylsulfonyl)benzamide.

## Revendications

1. Procédé de préparation de dérivés ortho-alkylés de l'acide benzoïque de formule I 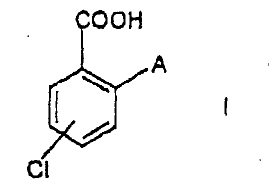 dans laquelle
A est un radical alkyle ayant de 1 à 4 atomes de C,
**caractérisé en ce qu'**un bromure d'aryle de formule II 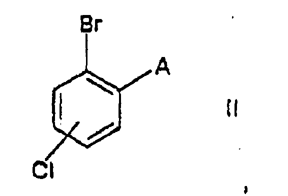 dans laquelle A est tel que défini dans la formule I,
est réagi avec un composé d'organolithium secondaire ou tertiaire et CO₂.

2. Procédé selon la revendication 1, **caractérisé en ce qu'**un composé d'organolithium secondaire choisi parmi le groupe : sec-butyllithium, isopropyllithium, sec-amyllithium, 4-heptyllithium, cyclopropyllithium ou cyclohexyllithium, ou un composé d'organolithium tertiaire choisi parmi le groupe : tert-butyllithium, tert-amyllithium, triéthylméthyllithium, 1-méthylcyclopentyllithium ou adamantyllithium, est employé.

3. Procédé selon la revendication 1 ou 2 pour la préparation de l'acide 4-chloro-2-méthylbenzoïque, **caractérisé en ce que** du 2-bromo-5-chloro-toluène est employé.

4. Procédé selon les revendications 1 à 3, **caractérisé en ce que** la réaction est réalisée à des températures comprises entre -100° et +50°C et le produit de réaction est précipité par l'addition d'un acide.

5. Procédé selon les revendications 1 à 4, **caractérisé en ce que** la réaction est réalisée dans un solvant inerte choisi parmi le groupe : diéthyl éther, méthyl tert-butyl éther, tétrahydrofurane, dioxane, toluène, hexane, éther de pétrole ou des mélanges de ceux-ci.

6. Procédé selon les revendications 1 à 5, **caractérisé en ce que** le bromure d'aryle de formule II est présenté dans un solvant inerte, le composé d'organolithium secondaire ou tertiaire est ajouté, ce mélange réactionnel est introduit goutte-à-goutte dans un solvant saturé en CO₂ et saturé de nouveau en CO₂.

7. Utilisation du procédé selon la revendication 3, pour la préparation de l'acide 4-chloro-2-méthylbenzoïque dans la synthèse de N-diaminométhylène-2-méthyl-4-(1-pyrrolyl)-5-méthylsulfonylbenzamide.

8. Procédé de préparation de N-diaminométhylène-2-méthyl-4-(1-pyrrolyl)-5-méthylsulfonylbenzamide, **caractérisé en ce que**
dans l'étape a) du 2-bromo-5-chlorotoluène est réagi avec un composé d'organolithium secondaire ou tertiaire et du CO₂ pour donner l'acide 4-chloro-2-méthylbenzoïque,
dans l'étape b) l'acide 4-chloro-2-méthylbenzoïque est réagi avec de l'acide chlorosulfonique, du sulfite de sodium et de l'iodure de méthyle pour donner l'acide 2-méthyl-4-chloro-5-méthylsulfonylbenzoïque,
dans l'étape c) l'acide 2-méthyl-4-chloro-5-méthylsulfonylbenzoïque est réagi avec de la benzylamine pour donner l'acide 4-benzylamino-5-méthylsulfonyl-2-méthylbenzoïque,
dans l'étape d) l'acide 4-benzylamino-5-méthylsulfonyl-2-méthylbenzoïque est estérifié par un alcool pour donner l'ester de l'acide 4 -benzylamino-5-méthylsulfonyl-2-méthylbenzoïque correspondant,
dans l'étape e) l'ester de l'étape d) est réduit en ester de l'acide 4-amino-5-méthylsulfonyl-2-méthylbenzoïque correspondant,
dans l'étape f) l'ester de l'acide 4-amino-5-méthylsulfonyl-2-méthylbenzoïque est réagi avec du diméthoxytétrahydrofurane pour donner l'ester de l'acide 2-méthyl-4-(1-pyrrolyl)-5-méthylsulfonylbenzoïque, et
dans l'étape g) l'ester de l'acide 2-méthyl-4-(1-pyrrolyl)-5-méthylsulfonylbenzoïque est réagi avec de la guanidine pour donner le N-diaminométhylène-2-méthyl-4-(1-pyrrolyl)-5-méthylsulfonylbenzamide.

9. Utilisation du procédé selon la revendication 3, pour la préparation de l'acide 4-chloro-2-méthylbenzoïque dans la synthèse du N-diaminométhylène-2-méthyl-4, 5-di(méthylsulfonyl)benzamide.

10. Procédé de préparation de N-diaminométhylène-2-méthyl-4,5-di(méthylsulfonyl)benzamide, **caractérisé en ce que**
dans l'étape a) du 2-bromo-5-chlorotoluène est réagi avec un composé d'organolithium secondaire ou tertiaire et du CO₂ pour donner l'acide 4-chloro-2-méthylbenzoïque,
dans l'étape b) l'acide 4-chloro-2-méthylbenzoïque est réagi avec de l'acide chlorosulfonique, du sulfite de sodium et de l'iodure de méthyle pour donner l'acide 2-méthyl-4-chloro-5-méthylsulfonylbenzoïque,
dans l'étape c) l'acide 2-méthyl-4-chloro-5-méthylsulfonylbenzoïque est réagi avec du méthylthiolate de sodium, et ensuite avec un oxydant pour donner l'acide 2-méthyl-4,5-di(méthylsulfonyl)benzoïque,
dans l'étape d) l'acide 2-méthyl-4,5-di(méthylsulfonyl)benzoïque est réagi avec du chlorure de thionyle pour donner le chlorure de 2-méthyl-4,5-di(méthylsulfonyl)benzoyle, et
dans l'étape e) le chlorure de 2-méthyl-4,5-di(méthylsulfonyl)benzoyle est réagi avec du chlorure de guanidinium pour donner le N-diaminométhylène-2-méthyl-4,5-di(méthylsulfonyl)benzamide.
